(19) Europäisches Patentamt
European Patent Office
Office européen des brevets

(11) **EP 4 193 922 A1**

(12) **EUROPEAN PATENT APPLICATION**
published in accordance with Art. 153(4) EPC

(43) Date of publication:
**14.06.2023 Bulletin 2023/24**

(21) Application number: **22810889.0**

(22) Date of filing: **28.02.2022**

(51) International Patent Classification (IPC):
***A61B 5/11*** *(2006.01)*

(52) Cooperative Patent Classification (CPC):
**A61B 5/11**

(86) International application number:
**PCT/JP2022/008153**

(87) International publication number:
**WO 2022/249608 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.05.2021 JP 2021090235**

(71) Applicants:
• **OSAKA UNIVERSITY**
**Suita-shi**
**Osaka 565-0871 (JP)**
• **Orphe Inc.**
**Tokyo, 151-0053 (JP)**

(72) Inventors:
• **NAKATA, Ken**
**Suita-shi, Osaka 565-0871 (JP)**
• **OGASAWARA, Issei**
**Suita-shi, Osaka 565-0871 (JP)**
• **KIKUKAWA, Yuya**
**Tokyo 151-0053 (JP)**
• **KOBAYASHI, Asumi**
**Tokyo 151-0053 (JP)**
• **UNO, Yuki**
**Suita-shi, Osaka 565-0871 (JP)**

(74) Representative: **Thompson, Trevor George**
**Karssen Limited**
**Suite LP49781**
**20-22 Wenlock Road**
**London N1 7GU (GB)**

(54) **GROUND-REACTION-FORCE-INDEX ESTIMATION SYSTEM, GROUND-REACTION-FORCE-INDEX ESTIMATION METHOD, AND GROUND-REACTION-FORCE-INDEX ESTIMATION PROGRAM**

(57) A floor reaction force index estimation system, a floor reaction force index estimation method, and a floor reaction force index estimation program that compute floor reaction force index at low computation costs are provided. A floor reaction force index estimation system, wherein a footwear includes a wearable sensor that measures triaxial acceleration and/or triaxial angular velocity and a computation unit that computes a floor reaction force indices by multiple regression analysis from values computed from the measurement results to be used as explanatory variables, and an external device includes a controller that evaluates walking and running motions for the user on the basis of the measurement results by the wearable sensor received from the footwear, the computed floor reaction force indices, and the determination reference information, and a presentation unit that presents the results of the evaluation of the running motion, and in the multiple regression analysis, the floor reaction force as a response variable is computed using a multiple regression equation in which a partial regression coefficient at a time when the results computed from the measurement results are used as explanatory variables is computed in advance.

FIG. 8

**Description**

BACKGROUND OF THE INVENTION

1. Field of the Invention

The present invention relates to a floor reaction force index estimation system, a floor reaction force index estimation method, and a floor reaction force index estimation program. More specifically, the present invention relates to a floor reaction force index estimation system, a floor reaction force index estimation method, and a floor reaction force index estimation program using a wearable sensor.

2. Description of Related Art

The floor reaction force index that is computed from the floor reaction force that the foot receives from the floor when the foot lands on the floor during walking or running is directed to an indicator of risk and performance of disability during walking or running (see, for example, non-patent document 1). The floor reaction force indices may include, for example, the loading rate, the kicking force, braking force integrated value (braking impulse), and acceleration force integrated value (acceleration impulse).

The floor reaction force index is computed from the change over time of the vertical and horizontal components of the floor reaction force relative to the floor surface during walking or running for one step, i.e., from a time when the foot lands on the floor to a time when the foot leaves the floor during walking or running. The loading rate is directed to a value computed from the angle of initial standing in the vertical component of the floor reaction force, and corresponds to an index of how rapidly the foot is loaded when the foot lands on the floor. The kicking force is directed to a peak value in the vertical component of the floor reaction force and indicates the force generated by kicking. The braking force integrated value is directed to an integral value of the rearward component of the floor reaction force, and corresponds to a braking component by the foot during walking or running. The acceleration force integrated value is directed to an integral value of the forward component of the floor reaction force, and corresponds to an acceleration component by the foot during walking or running.

The floor reaction force is measured by placing a board or sheet floor reaction force meter on the floor and walking or running on the floor reaction force meter. Since the measurement of the floor reaction force using a floor reaction force meter costs high and the measurement environment is limited to a location where the floor reaction force meter is installed, methods for computing the floor reaction force indices using low cost and compact wearable sensors (see, for example, patent documents 1 and 2) have been developed in recent years.

In patent document 2, the floor reaction force is computed on the basis of the output value of the pressure sensor, which value corresponds to the force loaded on the pressure sensor attached to the backside of the footwear that contacts the floor. However, this method limits the location of the sensors to be attached, and furthermore, sensors attached to the soles of the feet may interfere with natural walking and running movements. In contrast, in patent document 1, motion sensors are used as wearable sensors.

Prior Art Documents

Patent Documents

Patent Document 1: Japanese Laid-Open Patent Publication No. 2018-143412
Patent Document 2: Japanese Laid-Open Patent Publication No. 2008-298486 Non-Patent Documents
Non-Patent Document 1: A. A. Zadpoor, et al., Clinical Biomechanics 26, pp. 23 to 28

SUMMARY OF THE INVENTION

Although deep learning is a major method for computing floor reaction force from acceleration and angular velocity obtained from motion sensors, this method needs a considerable number of input variables and costs relatively computationally high. Accordingly, it is not easily implemented in a computing device built into a wearable sensor, in which computational resources are limited.

An object of the present invention is to provide a floor reaction force index estimation system, a floor reaction force index estimation method, and a floor reaction force index estimation program that compute floor reaction force indices at low computational costs on the basis of data obtained from wearable sensors in light of the above problems.

A summary of the first aspect of the present invention is directed to a floor reaction force index estimation system including footwear; and an external device connected to the footwear via a wireless line. The footwear includes: a wearable sensor, which measures at least one of triaxial acceleration and triaxial angular velocity, a first storage, which stores physical feature amount in advance, and a computation unit, which computes values to be used as explanatory variables from measurement results by the wearable sensor to compute floor reaction force and floor reaction force indices by multiple regression analysis from the values to be used as explanatory variables computed from the measurement results by the wearable sensor. The external device includes: a second storage, which stores determination reference information as information for determining walking and running motion of a user from the floor reaction force indices, a controller, which evaluates the walking and running motion for the user on the basis of the measurement results by the wearable sensor received from the footwear and the computed floor reaction force indices, and the determination reference information, and a presentation unit, which presents results of the evaluation of the running motion. In the multiple regression analysis, the floor reaction force as a response variable is computed using a multiple regression equation in which a partial regression coefficient at a time when the results computed from the measurement results are used as explanatory variables is computed in advance.

In the first aspect of the present invention, the values used as explanatory variables computed from the measurement results may correspond to at least one of the feature amount of the waveform data of the triaxial angular velocity, and the feature amount of the waveform data of the triaxial angular velocity.

In the first aspect of the present invention, the feature amount of the waveform data of the triaxial acceleration may include at least one of a global maximum value, a global minimum value, an average value, a local maximum value, a local minimum value, and a number of local maximum/minimum values of the waveform data of the triaxial acceleration, and the feature amount of the waveform data of the triaxial angular velocity may include at least one of a global maximum value, a global minimum value, an average value, a local maximum value, a local minimum value, and a number of local maximum/minimum values of the waveform data of the triaxial angular velocity.

In the first aspect of the present invention, the values used as explanatory variables computed from the measurement results may include at least one of the step speed (cadence), walking or running speed (speed), the angle at which the foot lands (strike angle), foot velocity (velocity), the height of the foot at the time of walking or running (foot height), and foot position (distance).

In the first aspect of the present invention, the values used as explanatory variables computed from the measurement results may include a physical feature amount.

In the first aspect of the present invention, the physical feature amount may include at least one of the height, weight, or foot length of the user.

In the first aspect of the present invention, the values used as explanatory variables computed from the measurement results may include walking and running conditions.

In the first aspect of the present invention, the walking/running conditions may include at least one of the slope angle of the ground, the hardness of the material of the ground, the friction coefficient of the material of the ground the hardness of the material of the footwear, and the coefficient of friction of the material of the footwear.

In the first aspect of the present invention, the external device may further include an input unit, and the physical feature amount may be entered through the input unit and transmitted from the external device to the footwear.

In the first aspect of the present invention, the external device may further include an input unit, and the walking/running conditions may be entered through the input unit and transmitted from the external device to the footwear.

In the first aspect of the present invention, the second storage may store the floor reaction force indices, the measurement results, and the changes of each of the floor reaction force indices and the measurement results over time.

A summary of the second aspect of the present invention is directed to a floor reaction force index estimation method including the steps of: detecting at least one of triaxial acceleration and triaxial angular velocity; computing values to be used as explanatory variables from at least one of the detected triaxial acceleration and triaxial angular velocity; computing floor reaction force and floor reaction force indices by multiple regression analysis from the computed values to be used as explanatory variables; computing the floor reaction force and floor reaction force indices by multiple regression analysis from results computed from measurement results for evaluating walking and running motion of a user on the basis of the computed floor reaction force indices and determination reference information as information for determining the walking and running motion of the user from the floor reaction force indices; and presenting results of the evaluation of the running motion. In the step of computing the floor reaction force and floor reaction force indices by the multiple regression analysis, the floor reaction force and floor reaction force indices are computed using a multiple regression equation in which a partial regression coefficient at a time when the results computed from the measurement results are used as explanatory variables is computed in advance.

In the second aspect of the present invention, the values used as explanatory variables computed from the measurement results may correspond to at least one of the feature amount of the waveform data of the triaxial angular velocity, and the feature amount of the waveform data of the triaxial angular velocity.

In the second aspect of the present invention, the feature amount of the waveform data of the triaxial acceleration

may include at least one of a global maximum value, a global minimum value, an average value, a local maximum value, a local minimum value, and a number of local maximum/minimum values of the waveform data of the triaxial acceleration, and the feature amount of the waveform data of the triaxial angular velocity may include at least one of a global maximum value, a global minimum value, an average value, a local maximum value, a local minimum value, and a number of local maximum/minimum values of the waveform data of the triaxial angular velocity.

In the second aspect of the present invention, the values used as explanatory variables computed from the measurement results may include at least one of the step speed (cadence), walking or running speed (speed), the angle at which the foot lands (strike angle), foot velocity (velocity), the height of the foot at the time of walking or running (foot height), and foot position (distance).

In the second aspect of the present invention, the values used as explanatory variables computed from the measurement results may include a physical feature amount.

In the second aspect of the present invention, the physical feature amount may include at least one of the height, weight, and foot length of the user.

In the second aspect of the present invention, the values used as explanatory variables computed from the measurement results may include walking/running conditions.

In the second aspect of the present invention, the walking/running conditions may include at least one of the slope angle of the ground, the hardness of the material of the ground, the friction coefficient of the material of the ground, the hardness of the material of the footwear, and the coefficient of friction of the material of the footwear.

A summary of the third aspect of the present invention is directed to a floor reaction force index estimation program causing a computer to execute the functions of detecting at least one of triaxial acceleration and triaxial angular velocity; computing values to be used as explanatory variables from at least one of the detected triaxial acceleration and triaxial angular velocity; computing floor reaction force and floor reaction force indices by multiple regression analysis from the computed values to be used as explanatory variables; computing the floor reaction force and floor reaction force indices using multiple regression equation in which results computed from measurement results, a feature amount of waveform data of the measurement results, and a physical feature amount are used as explanatory variables, wherein the results are computed from the measurement results for evaluating walking and running motion for a user on the basis of the computed floor reaction force indices and determination reference information as information for determining the walking and running motion of the user from the floor reaction force indices; and presenting results of the evaluation of the running motion.

In the third aspect of the present invention, the values used as explanatory variables computed from the measurement results may correspond to at least one of the feature amount of the waveform data of the triaxial angular velocity, and the feature amount of the waveform data of the triaxial angular velocity.

In the third aspect of the present invention, the feature amount of the waveform data of the triaxial acceleration may include at least one of a global maximum value, a global minimum value, an average value, a local maximum value, a local minimum value, and a number of local maximum/minimum values of the waveform data of the triaxial acceleration, and the feature amount of the waveform data of the triaxial angular velocity may include at least one of a global maximum value, a global minimum value, an average value, a local maximum value, a local minimum value, and a number of local maximum/minimum values of the waveform data of the triaxial angular velocity.

In the third aspect of the present invention, the values used as explanatory variables computed from the measurement results may include at least one of the step speed (cadence), walking or running speed (speed), the angle at which the foot lands (strike angle), foot velocity (velocity), the height of the foot at the time of walking or running (foot height), and foot position (distance).

In the third aspect of the present invention, the values used as explanatory variables computed from the measurement results may include a physical feature amount.

In the third aspect of the present invention, the physical feature amount may include at least one of the height, weight, or foot length of the user.

In the third aspect of the present invention, the values used as explanatory variables computed from the measurement results may include walking/running conditions.

In the third aspect of the present invention, the walking/running conditions may include at least one of the slope angle of the ground, the hardness of the material of the ground, the friction coefficient of the material of the ground, the hardness of the material of the footwear, and the coefficient of friction of the material of the footwear.

The present invention is capable of providing a floor reaction force index estimation system, a floor reaction force index estimation method, and a floor reaction force index estimation program that compute floor reaction force indices at low computational costs on the basis of data obtained from wearable sensors.

BRIEF DESCRIPTION OF THE DRAWINGS

FIG. 1 is a schematic diagram illustrating a floor reaction force index estimation system according to an embodiment of the present invention,

FIG. 2 is a block diagram illustrating an example of a configuration of a module according to an embodiment,

FIG. 3 is a block diagram illustrating an example of a configuration of an external device according to an embodiment,

FIG. 4 is a graph showing a relationship between actually measured values and estimated values of a loading rate,

FIG. 5 is a graph showing a relationship between actually measured values and estimated values of kicking force,

FIG. 6 is a graph showing a relationship between actually measured values and estimated values of braking force integrated values,

FIG. 7 is a graph showing a relationship between actually measured values and estimated values of acceleration force integrated values, and

FIG. 8 is a flowchart illustrating an operation of the floor reaction force index estimation system according to an embodiment.

DETAILED DESCRIPTION OF EMBODIMENTS

Next, an embodiment of the present invention will be described with reference to the drawings. In the description of the drawings according to the embodiment, the same or similar reference numerals are assigned to the same or similar components. In addition, it is assumed that the drawings each other may include portions that differ from each other in their relationship.

Also, the embodiments illustrate examples of devices and methods for embodying the technical concept of the present invention, and the technical concept of the present invention does not specify the configuration of the components to the following, for example. The technical concept of the present invention may be modified in various ways within the technical scope defined by the claims.

Embodiments

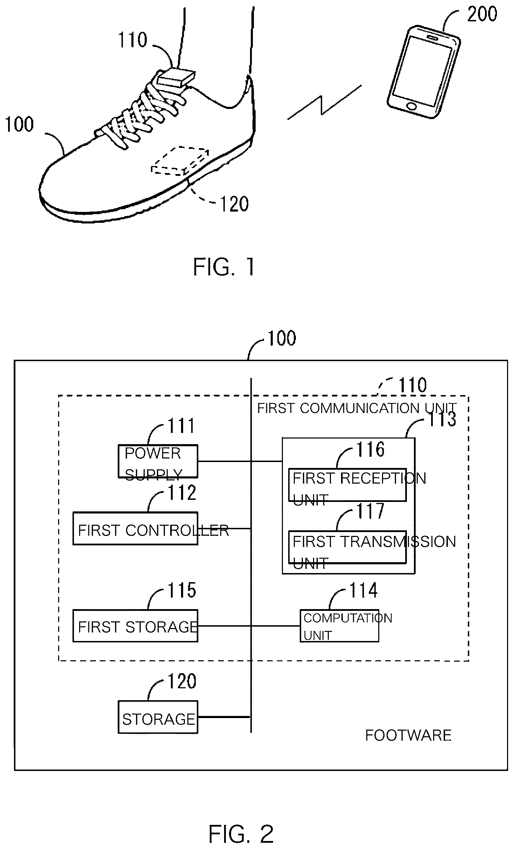

The floor reaction force index estimation system according to the present embodiment will be described with reference to FIGS. 1 through 3. As shown in FIG. 1, the floor reaction force index estimation system 10 according to the present embodiment includes footwear 100 and an external device 200. The footwear 100 includes a module 110 and a sensor 120 each attached to the footwear 100. The module 110 and the sensor 120 are connected to each other via a bus.

The footwear 100 is worn by a user on the foot, which may include running shoes, sneakers, leather shoes, and sandals, for example. In FIG. 1, although the module 110 is located on the top of the footwear 100 and the sensor 120 is located on the bottom of the footwear 100, the mounting positions of the module 110 and the sensor 120 on the footwear 100 are not limited to the locations if the sensor 120 is allowed to measure triaxial acceleration and/or triaxial angular velocity and fails to interfere with the walking and running. Here, the triaxial acceleration and/or triaxial angular velocity correspond to the acceleration in the triaxial direction, i.e., a direction in two axes perpendicular to each other in the horizontal plane and an axis perpendicular to the two axes, and the angular velocity around each of the three axes.

The sensor 120 is directed to a sensor that detects the movement of the footwear 100 as the user wears the footwear 100, and walks and runs. In this embodiment, the sensor 120 corresponds to a sensor that detects triaxial acceleration and/or triaxial angular velocity. The sensor 120 may have, for example, an acceleration sensor that detects triaxial acceleration and an angular velocity sensor that detects triaxial angular velocity.

As shown in FIG. 2, the module 110 includes a power supply 111, a first controller 112, a first communication unit 113, a computation unit 114, and a first storage 115.

The power supply 111 supplies power to the module 110 and the sensor 120.

The first controller 112 corresponds to a processor that controls the module 110 and the sensor 120. The first controller 112 controls the module 110 and the sensor 120 by executing the control program stored in the first storage 115.

The first communication unit 113 corresponds to a communication interface that communicates with the external device 200. The first communication unit 113 includes a first reception unit 116 and a first transmission unit 117. The first communication unit 113 communicates with the external device 200 by wireless communication. The first communication unit 113 may communicate with the external device 200 by any telecommunications standard if it is capable of communicating with the external device 200.

The first reception unit 116 receives, from the external device 200, the start and end of measurement by the sensor 120, information related to the physical feature amount and/or walking/running conditions of the user used in computing the floor reaction force index by multiple regression analysis by the computation unit 114, and causes the first storage 115 to store the physical feature amount and/or walking/running conditions of the user. Although the physical feature amount of the user corresponds to, for example, height, weight, foot length, and foot width, it may include any other physical feature amount as well. In the present embodiment, the physical feature amount of the user includes at least one of the height, weight, and foot length. Although the walking/running conditions correspond to, for example, the slope angle of the ground, the hardness and friction coefficient of the ground material, and the hardness and friction coefficient of the shoe material, they may include any other walking/running conditions as well.

The first transmission unit 117 transmits, to the external device 200, the status of the measurement by the sensor 120, information related to the floor reaction force index computed by the multiple regression analysis, and the results computed from the measurement results by the sensor 120. Although the results computed from the measurement results by the sensor 120 may correspond to, for example, the step speed (cadence), walking or running speed (speed), angle at which the foot lands (strike angle), foot velocity (velocity), foot height at a time of walking or running (foot height), and foot position (distance) of the user, they may also include any other indices.

The results computed from the measurement results by the sensor 120 may include the results obtained using the triaxial acceleration, the results obtained using the triaxial angular velocity, results obtained using the triaxial acceleration and the triaxial angular velocity, that are measured by the sensor 120. In the present embodiment, the results computed from the measurement results by the sensor 120 include at least one of the step speed (cadence), walking or running speed (speed), the angle at which the foot lands (strike angle), foot velocity (velocity), the height of the foot at a time of walking or running (foot height), and foot position (distance).

The computation unit 114 computes the values used as explanatory variables from the triaxial acceleration and/or triaxial angular velocity measured by the sensor 120. The values used as explanatory variables include the feature amount of the waveform data of the triaxial acceleration and/or triaxial angular velocity. Here, although the feature amount of the waveform data of the triaxial acceleration and/or triaxial angular velocity computed in the computation unit 114 corresponds to, for example, a global maximum value, global minimum value, average value, local maximum value, local minimum value, and a number of local maximum/minimum values of the triaxial acceleration and/or triaxial angular velocity, it may include any other feature amount.

The values used as explanatory variables may also include at least one of the cadence, speed, strike angle, velocity, foot height, and distance. The values used as explanatory variables may further include a physical feature amount of the user. The values used as explanatory variables may further include walking/driving conditions.

As described below, which values are used as the explanatory variables are determined depending on the floor reaction force index desired to be computed by the computation unit 114 when the floor reaction force and the floor reaction force index are computed using the multiple regression equation stored in the first storage 115 and the values to be used as explanatory variables. Also, when the floor reaction force indices to be computed are different from each other, the multiple regression equations and the partial regression coefficients of the multiple regression equations as well as the explanatory variables used to estimate the floor reaction force indices are different from each other as well. That is, the values used as explanatory variables, the multiple regression equations, and the partial regression coefficients of the multiple regression equations are selected according to the floor reaction force indices desired to be computed. The first storage 115 may store a table, for example, showing the correspondence between the floor reaction force indices to be computed and the corresponding explanatory variables, multiple regression equations, and partial regression coefficients of the multiple regression equations, and then the values to be used as explanatory variables, the multiple regression equations, and the partial regression coefficients of the multiple regression equations may be selected according to the floor reaction force indices desired to be computed with reference to the table.

In the present embodiment, the values computed in the computation unit 114 and used as explanatory variables are at least one of the cadence, speed, strike angle, velocity, foot Height, and distance. In the present embodiment, the feature amount of the waveform data of the triaxial acceleration and/or triaxial angular velocity includes at least one of the global maximum and minimum values of the triaxial acceleration. The feature amount of the waveform data of the triaxial acceleration and triaxial angular velocity may further include, for example, the global maximum and minimum values of the triaxial angular velocity.

Further, the computation unit 114 computes the first floor reaction force and the floor reaction force indices using the multiple regression equations stored in the storage 115 and the values used as explanatory variables. In this case, as described above, the first storage may store, for example, a table showing the correspondence between the floor reaction force indices to be computed and the corresponding explanatory variables, multiple regression equations, and partial regression coefficients of the multiple regression equations, and then the multiple regression equations stored in the first storage 115 and the values used as explanatory variables may be selected according to the floor reaction force indices desired to be computed. In the present embodiment, although the floor reaction force index in the computation unit 114 corresponds to loading speed, kicking force, braking force integrated values (braking impulse), and acceleration force integrated values (acceleration impulse), the floor reaction force index computed in the computation unit 114 may include any other floor reaction force indices if they may be computed from the floor reaction force.

The external device 200 may be in any form, e.g., a personal computer, and mobile terminal, if it is capable of transmitting, to the first reception unit 116, information indicating the start and end of measurement by the sensor 120, and the physical feature amount and walking/running conditions to perform multiple regression analysis in the computation unit 114, and receiving, from the first transmission unit 117, the start and end of measurement by the sensor 120 and the floor reaction force indices. For checking the evaluation of walking and running motions in real time with the user wearing the footwear 100 and performing measurements by the sensor 120, it may be desirable the external device 200 may be a portable terminal such as a mobile terminal.

The external device 200 may be in a form that can be attached to a portion of the body, such as a wristwatch or earphones, for example. If the external device 200 is shaped like a wristwatch, for example, the evaluation of walking and running motions may be checked on the display of the wristwatch-shaped external device 200 without interfering with the walking and running motions. If the external device 200 corresponds to, for example, an earphone, the evaluation of walking or running movements may be checked by voice.

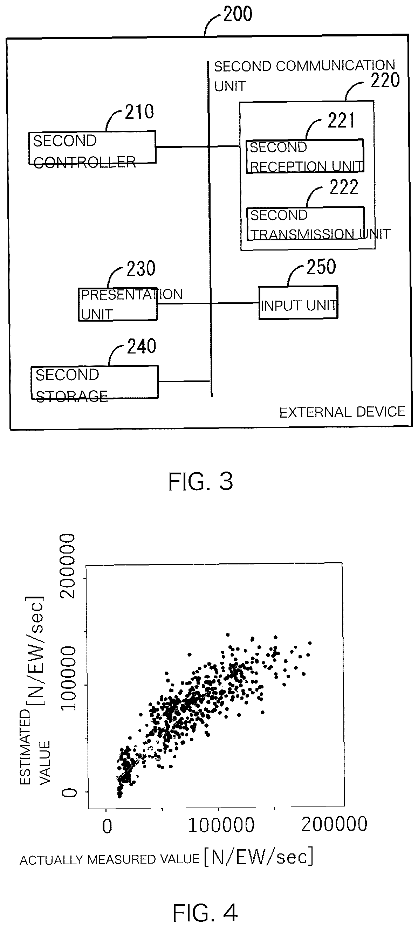

As shown in FIG. 3, the external device 200 includes a second controller 210, a second communication unit 220, a presentation unit 230, a second storage 240, and an input unit 250.

The second controller 210 corresponds to a processor that controls portions of the external device 200. Specifically, the second controller 210 controls the portions of the external device 200 by executing a control program stored in the second storage 240. The second controller 210 evaluates the walking and running motions of the user wearing the footwear 100 and walking or running on the basis of the received floor reaction force indices and the determination reference information stored in the second storage 240, and transmits the evaluation results to the presentation unit 230.

The determination reference information corresponds to information for determining the walking and running motions of the user on the basis of the floor reaction force indices. The loading rate is directed to an index of how rapidly the load is applied to the foot when the foot lands on the floor. For example, a threshold value for the loading rate may be set as determination reference information in advance, and if the loading rate is greater than the threshold value, the user may be alerted that the foot is overloaded. The braking force integrated value (braking impulse) corresponds to a braking component of the foot during walking and driving. A threshold value of the braking force integrated value is set in advance, and if the braking force integrated value is greater than the threshold value, the user may be determined to perform inefficient running and notified of that. In the same manner, the acceleration force integrated value (acceleration impulse) corresponds to acceleration component of foot during walking and running. A threshold value of the acceleration force integrated value is set in advance, and if the acceleration force integrated value is greater than the threshold value, it may be determined that a large propulsive force has been obtained, and the user may be notified of that.

The second communication unit 220 corresponds to a communication interface that communicates with the footwear 100. The second communication unit 220 includes a second reception unit 221 and a second transmission unit 222. The second communication unit 220 communicates with the footwear 100 via wireless communication. The second communication unit 220 may communicate by any telecommunications standard if it is capable of communicating with the footwear 100.

The presentation unit 230 presents the received floor reaction force index. The presentation unit 230 may, in addition to or in place of the floor reaction force index, present alert and evaluation results to a user using the footwear 100 on the basis of the evaluation results transmitted from the second controller 210. The presentation unit 230 is capable of presenting the alert and evaluation results to the user in text, pictorial or audio form. In addition, the presentation unit 230 may present information notifying the walking or running conditions of the user such as step speed (cadence), and walking or running speed (speed). Input by the user through the input unit 250 may specify which information is to be presented.

In addition to the control program, determination reference information, physical feature amount of the user, and walking/running conditions, the second storage 240 may store the floor reaction force indices computed by multiple regression analysis, results obtained from the measurement by the sensor 120, i.e., for example, the step speed (cadence) of the user, the walking or running speed (speed), the angle at which the foot lands (strike angle), the velocity of the foot (velocity), the height of the foot at a time when the user is walking or running (foot height), and the position of the foot (distance) with the date and time of the measurement by the sensor 120. The second controller 210 is capable of determining the effectiveness of the training of the user, i.e., walking or running training, for example, on the basis of the changes over time in the floor reaction force index and measurement results.

The input unit 250 receives inputs from the user. The user inputs information associated with the physical feature amount of the user used in computing the floor reaction force index by multiple regression analysis in the computation unit 114, i.e., height, weight, and foot length, as well as walking/running conditions through the input unit 250. The second storage 240 stores the physical feature amount of the user and the walking/running conditions entered through the input unit 250.

Although FIG. 1 shows an example in which the footwear 100 is connected via wireless communication to one external device 200, it may be connected to multiple external devices 200. For example, if the footwear 100 is connected to a personal computer and a mobile terminal as external devices via wireless communication, and the physical feature amount of the user are entered from the personal computer, the mobile terminal does not need to include an input unit to allow the mobile terminal to be compact.

In addition, the floor reaction force indices stored in the second storage 240 and the data of the change of the measurement results over time may be stored, for example, in a storage that the personal computer includes in place of the second storage 240 with the footwear 100 connected via wireless communication to the personal computer and mobile terminal as external devices.

Next, a method for obtaining a multiple regression equation used to compute the floor reaction force in the computation unit 114 will be described.

A multiple regression equation is generally directed to an equation in which the response variable is expressed as a function of one or more explanatory variables. It may be expressed as a linear function of one or more explanatory variables, or a polynomial equation of the quadratic or higher degree, for example. In this embodiment, the following equation is used as the multiple regression equation.

Equation 1

$$Y_i = \beta_0 + \beta_1 x_{i1} + \beta_2 x_{i2} + \cdots + \beta_p x_{ip} + E_i$$

Here, $Y_i$ is a response variable, which in this embodiment is the floor reaction force, $\beta_0$ and $\beta_n$ are partial regression coefficients, $x_{in}$ is an explanatory variable, and $E_i$ is an error term (where n is an integer from 1 to p). The explanatory variables are directed to, in the present embodiment, the feature amount of the waveform data of the triaxial acceleration and triaxial angular velocity, the results computed from the triaxial acceleration and triaxial angular velocity measured by the sensor 120, the physical feature amount of the user, and the walking/running conditions. In the present embodiment, at least one of the maximum value of acceleration waveform data and the minimum value of acceleration waveform data is used as a feature value of wave data of the triaxial acceleration and/or triaxial angular velocity. In the present embodiment, as a result computed from the triaxial acceleration and triaxial angular velocity, at least one of the step speed (cadence), walking or running speed (speed), the angle at which the foot lands on the floor (strike angle), foot velocity (velocity), the height of the foot at a time of walking or running (foot height), and foot position (distance) is used. In the present embodiment, as the physical feature amount of the subject, at least one of the height, weight, and foot length of the subject is used. As the walking/running conditions, in the present embodiment, at least one of the slope angle of the ground, the hardness and friction coefficients of the ground material, and the hardness and friction coefficients of the shoe material is used.

To obtain the partial regression coefficients of the above equation, the floor reaction force was measured and the triaxial acceleration and triaxial angular velocity were measured by the sensor 12. Specifically, the subject walked and ran on the floor reaction force meter with the footwear 100 worn, and the floor reaction force was measured and the triaxial acceleration and triaxial angular velocity were measured simultaneously by the sensor 12. From the triaxial acceleration and triaxial angular velocity obtained from the measurements by the sensor 120, the values to be used as explanatory variables, i.e., at least one of the cadence, speed, strike angle, velocity, foot height, and distance, and the feature amount of the waveform data of the triaxial acceleration and triaxial angular velocity, i.e., at least one of the global maximum value of the waveform data of the acceleration and the global minimum value of the waveform data of the acceleration, were computed, and then from the floor reaction force value obtained from the measurements by the floor reaction force meter, at least one of the computed cadence, speed, strike angle, velocity, foot height, and distance, at least one of the global maximum value of the waveform data of the acceleration and the global minimum value of the waveform data of the acceleration, and at least one of the height, weight, and foot length of the subject, the multiple regression equation was obtained by computing the partial regression coefficients on the basis of the least squares method.

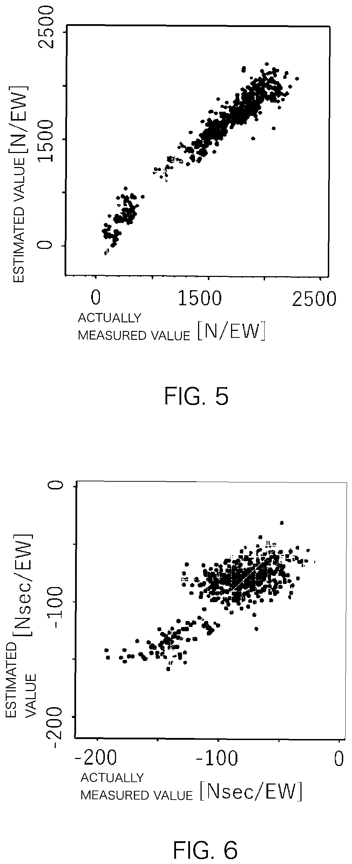

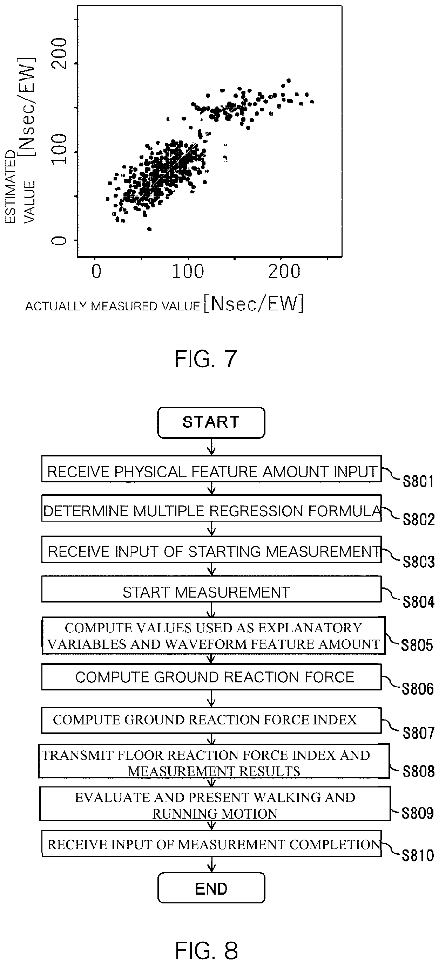

FIGS. 4 to 7 show values computed from the actually measured values of the floor reaction force such as the loading speed (loading rate), kicking force (kicking force), braking force integrated value (braking impulse), and acceleration force integrated value (acceleration impulse) as the floor reaction force indices computed from the floor reaction force, and the estimated values of the floor reaction force obtained from the multiple regression equation. FIG. 4 shows the loading speed, FIG. 5 shows the kicking force, FIG. 6 shows the braking force integrated value, and FIG. 7 shows the acceleration force integrated value. The values shown in FIGS. 4 to 7 represent the results of measurements performed on 20 subjects. Regarding the shading of the dots in data, the data from the same subject is of the same shading.

Table 1 below shows the coefficients $R^2$ of determination and the average value $R_{ave}$ of the multiple correlation coefficients computed from the values shown in FIGS. 4 to 7.

Table 1

|  | determination coefficient $R^2$ | average value $R_{ave}$ of multiple correlation coefficients |
|---|---|---|
| Loading Rate | 764 | 0.831 |
| Kicking Force | 0.939 | 0.943 |

(continued)

|  | determination coefficient $R^2$ | average value $R_{ave}$ of multiple correlation coefficients |
|---|---|---|
| Braking Impulse | 0.672 | 0.779 |
| Acc Impulse | 0.756 | 0.846 |

The coefficients $R^2$ of determination and the average value $R_{ave}$ of the multiple correlation coefficients take values between 0 and 1, respectively, with values closer to 1 indicating that the measured and estimated values are closer to each other. The coefficients $R^2$ of determination and the average values $R_{ave}$ of the multiple correlation coefficients shown in table 1 above are all greater than or equal to about 7, and the multiple regression equation used in the present embodiment is determined to be reasonable.

Next, an example of the operation of the floor reaction force index estimation system 10 will be described with reference to FIG. 8, when the user performs measurement by the sensor 120 with the footwear 100 worn to evaluate the walking or running of the user. It is assumed that the partial regression coefficients in the multiple regression equation have already been determined, the user is wearing the footwear 100, and the power supply 111 is turned on. The user is walking or running, or the user starts walking or running after the measurement by the sensor 120 starts.

In step S801, the input unit 250 receives input of physical feature amount and walking/running conditions from the user, and the physical feature amount and walking/running conditions are transmitted from the second communication unit 220 to the first reception unit 116.

In step S802, the physical feature amount and walking/running conditions received by the first reception unit 116 are stored in the first storage 115, and a multiple regression equation is determined, which is used in computing the floor reaction force indices on the basis of the multiple regression analysis by the computation unit 114.

In step S803, the input unit 250 receives an input from the user to start measurement by the sensor 120, and an input signal to start measurement is transmitted from the second communication unit 220 to the first reception unit 116.

In step S804, the measurement by the sensor 120 is started.

In step S805, the computation unit 114 computes the values to be used as explanatory variables from the triaxial acceleration and triaxial angular velocity measured by the sensor 120, i.e., at least one of the step speed (cadence), the speed of walking or running (speed), the angle at which the foot lands on the floor (strike angle), the velocity of the side of the foot (velocity), the height of the foot at the time of walking or running (foot height), and the position of the foot (distance), and the feature amount of the waveform data of the triaxial acceleration and triaxial angular velocity, i.e. at least one of the global maximum value of the waveform data of the acceleration and the global minimum value of the waveform data of the acceleration.

In step S806, the computation unit 114 computes the floor reaction force on the basis of at least one of the cadence, speed, strike angle, velocity, foot height, and distance computed in step S805, at least one of the global maximum and minimum values of the waveform data of the triaxial acceleration measured by the sensor 120, at least one of the height, weight, and foot length, and at least one of the slope angle of the ground, the hardness and friction coefficient of the ground material, and the hardness and friction coefficient of the shoe material.

In step S807, the floor reaction force indices (loading rate, kicking force, braking force integrated values, and acceleration force integrated values) are computed.

In step S808, the computed floor reaction force index and the results obtained from the measurements by the sensor 120 are transmitted from the first transmission unit 117 to the second communication unit 220 and stored in the second storage 240.

In step S809, the second controller 210 evaluates the walking and running motion of the user on the basis of the floor reaction force index and the determination reference information stored in the second storage 240, and transmits the evaluation results to the presentation unit 230.

In step S810, the input unit 250 receives an input from the user to terminate the measurement by the sensor 120, and the measurement by the sensor 120 is terminated.

As described above, the present invention uses data obtained from inexpensive motion sensors to allow floor reaction force indicators that are conventionally obtained using floor reaction force meters to be estimated. This allows the computation of the floor reaction force indices in any measurement environment, which has been difficult with conventional measurement methods. In addition, in comparison to the conventional estimation methods such as those using machine learning, this method is less computationally expensive, facilitating implement in microcomputers with built-in sensors that have limited computational resources, and achieving high versatility. The low computational costs also facilitate real-time processing. Real-time feedback of information during walking and running is effective in transforming the behavior of the person involved and can be a useful technology for, for example, runners seeking to improve or prevent disability and improve performance, and for patients and instructors associated with rehabilitation.

The above invention may include various embodiments that are not described here. Therefore, the technical scope of the present invention is defined only by the invention specific features recited in the claims which are reasonable from the above description.

Description of Reference Numerals

10: floor reaction force index estimation system
100: footwear
110: module
111: power supply
112: first controller
113: first communication unit
114: computation unit
115: storage
116: first reception unit
117: first transmission unit
120: sensor
200: external device
210: second controller
220: second communication unit
221: second reception unit
222: second transmission unit
230: presentation unit
240: second storage
250: input unit

**Claims**

1.    A floor reaction force index estimation system comprising footwear; and an external device connected to the footwear via a wireless line, wherein

the footwear includes:

a wearable sensor, which measures at least one of triaxial acceleration and triaxial angular velocity,
a first storage, which stores physical feature amount, and
a computation unit, which computes values to be used as explanatory variables from measurement results by the wearable sensor to compute floor reaction force and floor reaction force indices by multiple regression analysis from the values to be used as explanatory variables computed from the measurement results by the wearable sensor, and

the external device includes:

a second storage, which stores determination reference information as information for determining walking and running motion of a user from the floor reaction force indices,
a controller, which evaluates the walking and running motion for the user on the basis of the measurement results by the wearable sensor received from the footwear and the computed floor reaction force indices, and the determination reference information, and
a presentation unit, which presents results of the evaluation of the running motion, and

in the multiple regression analysis, the floor reaction force as a response variable is computed using a multiple regression equation in which a partial regression coefficient at a time when the results computed from the measurement results are used as explanatory variables is computed in advance.

2.    The floor reaction force index estimation system according to claim 1, wherein the values to be used as explanatory variables computed from the measurement results correspond to at least one of a feature amount of waveform data of the triaxial acceleration and a feature amount of waveform data of the triaxial angular velocity.

3. The floor reaction force index estimation system according to claim 2, wherein the feature amount of the waveform data of the triaxial acceleration includes at least one of a global maximum value, a global minimum value, an average value, a local maximum value, a local minimum value, and a number of local maximum/minimum values of the waveform data of the triaxial acceleration, and the feature amount of the waveform data of the triaxial angular velocity includes at least one of a global maximum value, a global minimum value, an average value, a local maximum value, a local minimum value, and a number of local maximum/minimum values of the waveform data of the triaxial angular velocity.

4. The floor reaction force index estimation system according to claim 2 or 3, wherein the values to be used as explanatory variables computed from the measurement results further include at least one of step speed (cadence), walking or running speed (speed), angle at which a foot lands (strike angle), speed of a side of the foot (velocity), foot height at a time of walking or running (foot height), and a foot position (distance).

5. The floor reaction force index estimation system according to any one of claims 2 to 4, wherein the values to be used as explanatory variables computed from the measurement results include a physical feature amount.

6. The ground reaction force index estimation system according to claim 5, wherein the physical feature amount includes at least one of height, weight, and foot length of the user.

7. The ground reaction force index estimation system according to any one of claims 2 to 6, wherein the values to be used as explanatory variables computed from the measurement results include walking/running conditions.

8. The ground reaction force index estimation system according to claim 7, wherein the walking/running conditions include at least one of slope angle of the ground, hardness of a material of the ground, friction coefficient of the material of the ground, hardness of a material of the footwear, and friction coefficient of the material of the footwear.

9. The ground reaction force index estimation system according to claim 5 or 6, wherein the external device further includes an input unit, and the physical feature amount is entered through the input unit and transmitted from the external device to the footwear.

10. The ground reaction force index estimation system according to claim 7 or 8, wherein the external device further includes an input unit, and the walking/running conditions are entered through the input unit and transmitted from the external device to the footwear.

11. The ground reaction force index estimation system according to any one of claims 1 to 10, wherein the second storage stores the ground reaction force indices, the measurement results, and changes of each of the ground reaction force indices and the measurement results over time.

12. A floor reaction force index estimation method comprising the steps of:

detecting at least one of triaxial acceleration and triaxial angular velocity;
computing values to be used as explanatory variables from at least one of the detected triaxial acceleration and triaxial angular velocity;
computing floor reaction force and floor reaction force indices by multiple regression analysis from the computed values to be used as explanatory variables;
computing the floor reaction force and floor reaction force indices by multiple regression analysis from results computed from measurement results for evaluating walking and running motion of a user on the basis of the computed floor reaction force indices and determination reference information as information for determining the walking and running motion of the user from the floor reaction force indices; and
presenting results of the evaluation of the running motion, wherein
in the step of computing the floor reaction force and floor reaction force indices by the multiple regression analysis, the floor reaction force and floor reaction force indices are computed using a multiple regression equation in which a partial regression coefficient at a time when the results computed from the measurement results are used as explanatory variables is computed in advance.

13. The floor reaction force index estimation method according to claim 12, wherein the values to be used as explanatory variables computed from the measurement results correspond to at least one of a feature amount of waveform data of the triaxial acceleration and a feature amount of waveform data of the triaxial angular velocity.

14. The floor reaction force index estimation method according to claim 13, wherein the feature amount of the waveform data of the triaxial acceleration includes at least one of a global maximum value, a global minimum value, an average value, a local maximum value, a local minimum value, and a number of local maximum/minimum values of the waveform data of the triaxial acceleration, and the feature amount of the waveform data of the triaxial angular velocity includes at least one of a global maximum value, a global minimum value, an average value, a local maximum value, a local minimum value, and a number of local maximum/minimum values of the waveform data of the triaxial angular velocity.

15. The floor reaction force index estimation method according to claim 13 or 14, wherein the values to be used as explanatory variables further include at least one of step speed (cadence), walking or running speed (speed), angle at which a foot lands (strike angle), speed of a side of the foot (velocity), foot height at a time of walking or running (foot height), and a foot position (distance).

16. The floor reaction force index estimation method according to any one of claims 13 to 15, wherein the values to be used as explanatory variables computed from the measurement results include a physical feature amount.

17. The ground reaction force index estimation method according to claim 16, wherein the physical feature amount includes at least one of height, weight, and foot length of the user.

18. The ground reaction force index estimation method according to any one of claims 13 to 17, wherein the values to be used as explanatory variables computed from the measurement results include walking/running conditions.

19. The ground reaction force index estimation method according to claim 18, wherein the walking/running conditions include at least one of slope angle of the ground, hardness of a material of the ground, friction coefficient of the material of the ground, hardness of a material of footwear, and friction coefficient of the material of the footwear.

20. A floor reaction force index estimation program causing a computer to execute the functions of

detecting at least one of triaxial acceleration and triaxial angular velocity;
computing values to be used as explanatory variables from at least one of the detected triaxial acceleration and triaxial angular velocity;
computing floor reaction force and floor reaction force indices by multiple regression analysis from the computed values to be used as explanatory variables;
computing the floor reaction force and floor reaction force indices using multiple regression equation in which results computed from measurement results are used as explanatory variables, wherein the results are computed from the measurement results for evaluating walking and running motion for a user on the basis of the computed floor reaction force indices and determination reference information as information for determining the walking and running motion of the user from the floor reaction force indices; and
presenting results of the evaluation of the running motion.

21. The floor reaction force index estimation program according to claim 20, wherein the values to be used as explanatory variables computed from the measurement results correspond to at least one of a feature amount of waveform data of the triaxial acceleration and a feature amount of waveform data of the triaxial angular velocity.

22. The floor reaction force index estimation program according to claim 21, wherein the feature amount of the waveform data of the triaxial acceleration includes at least one of a global maximum value, a global minimum value, an average value, a local maximum value, a local minimum value, and a number of local maximum/minimum values of the waveform data of the triaxial acceleration, and the feature amount of the waveform data of the triaxial angular velocity includes at least one of a global maximum value, a global minimum value, an average value, a local maximum value, a local minimum value, and a number of local maximum/minimum values of the waveform data of the triaxial angular velocity.

23. The floor reaction force index estimation program according to claim 21 or 22, wherein the values to be used as explanatory variables further include at least one of step speed (cadence), walking or running speed (speed), angle at which a foot lands (strike angle), speed of a side of the foot (velocity), foot height at a time of walking or running (foot height), and a foot position (distance).

24. The floor reaction force index estimation program according to any one of claims 21 to 23, wherein the values to be used as explanatory variables computed from the measurement results include a physical feature amount.

25. The ground reaction force index estimation program according to claim 24, wherein the physical feature amount includes at least one of height, weight, and foot length of the user.

26. The ground reaction force index estimation program according to any one of claims 21 to 25, wherein the values to be used as explanatory variables computed from the measurement results include walking/running conditions.

27. The ground reaction force index estimation program according to claim 26, wherein the walking/running conditions include at least one of slope angle of the ground, hardness of a material of the ground, friction coefficient of the material of the ground, hardness of a material of footwear, and friction coefficient of the material of the footwear.

FIG. 1

FIG. 2

FIG. 3

ACTUALLY MEASURED VALUE $[N/EW/sec]$

FIG. 4

FIG. 5

FIG. 6

FIG. 7

START

RECEIVE PHYSICAL FEATURE AMOUNT INPUT — S801

DETERMINE MULTIPLE REGRESSION FORMULA — S802

RECEIVE INPUT OF STARTING MEASUREMENT — S803

START MEASUREMENT — S804

COMPUTE VALUES USED AS EXPLANATORY VARIABLES AND WAVEFORM FEATURE AMOUNT — S805

COMPUTE GROUND REACTION FORCE — S806

COMPUTE GROUND REACTION FORCE INDEX — S807

TRANSMIT FLOOR REACTION FORCE INDEX AND MEASUREMENT RESULTS — S808

EVALUATE AND PRESENT WALKING AND RUNNING MOTION — S809

RECEIVE INPUT OF MEASUREMENT COMPLETION — S810

END

FIG. 8

## INTERNATIONAL SEARCH REPORT

International application No.

**PCT/JP2022/008153**

### A. CLASSIFICATION OF SUBJECT MATTER

*A61B 5/11*(2006.01)i
FI: A61B5/11 210

According to International Patent Classification (IPC) or to both national classification and IPC

### B. FIELDS SEARCHED

Minimum documentation searched (classification system followed by classification symbols)

A61B5/11

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2008-298486 A (KOCHI UNIV OF TECHNOLOGY) 11 December 2008 (2008-12-11) paragraphs [0023]-[0073], fig. 1-18 | 1, 11-12, 20 |
| A | | 2-10, 13-19, 21-27 |
| Y | JP 2016-150193 A (KOCHI PREFECTURAL PUBLIC UNIV CORP) 22 August 2016 (2016-08-22) paragraphs [0010]-[0089], fig. 1-6 | 1, 11-12, 20 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **17 March 2022** | **17 May 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**
Information on patent family members

International application No.

**PCT/JP2022/008153**

| Patent document cited in search report | | | Publication date (day/month/year) | Patent family member(s) | Publication date (day/month/year) |
|---|---|---|---|---|---|
| JP | 2008-298486 | A | 11 December 2008 | (Family: none) | |
| JP | 2016-150193 | A | 22 August 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018143412 A

- JP 2008298486 A

**Non-patent literature cited in the description**

- **A. A. ZADPOOR et al.** *Clinical Biomechanics,* vol. 26, 23-28